**Europäisches Patentamt**

**European Patent Office**

**Office européen des brevets**

(19)

(11) Veröffentlichungsnummer : **0 073 056**
**B1**

(12) **EUROPÄISCHE PATENTSCHRIFT**

(45) Veröffentlichungstag der Patentschrift :
21.01.87

(21) Anmeldenummer : 82107729.4

(22) Anmeldetag : 24.08.82

(51) Int. Cl.⁴ : **G 01 N 35/00,** G 01 N 33/52

(54) Analyseteststreifen und Verfahren zu seiner Herstellung.

(30) Priorität : 27.08.81 DE 3133826

(43) Veröffentlichungstag der Anmeldung :
02.03.83 Patentblatt 83/09

(45) Bekanntmachung des Hinweises auf die Patenterteilung : 21.01.87 Patentblatt 87/04

(84) Benannte Vertragsstaaten :
**AT BE CH DE FR GB IT LI LU NL SE**

(56) Entgegenhaltungen :
CH-A-   456 199
FR-A- 1 350 340
FR-A- 2 258 630
FR-A- 2 368 774
GB-A- 2 001 435
US-A- 3 932 133

(73) Patentinhaber : **Boehringer Mannheim GmbH**
**Sandhofer Strasse 116**
**D-6800 Mannheim 31 (DE)**

(72) Erfinder : **Poppe, Werner, Dr.**
**Magdeburger Strasse 3**
**D-6712 Bobenheim-Roxheim 2 (DE)**
Erfinder : **van Rijckevorsel, Rainer, Dipl.-Ing.**
**Freiburger Strasse 3**
**D-6831 Brühl (DE)**
Erfinder : **Ruppender, Uwe**
**Ifflandstrasse 7**
**D-6800 Mannheim (DE)**
Erfinder : **Macho, Heinz Kurt**
**Reiterweg 50**
**D-6800 Mannheim-Neuostheim (DE)**

**Beschreibung**

Die Erfindung betrifft ein Verfahren zum Herstellen eines Analyseteststreifens mit einem länglichen Träger, auf dem mindestens ein Testfeld befestigt ist, bei dem das Testfeldmaterial chargenweise in einer für eine Vielzahl von Teststreifen ausreichenden Menge hergestellt und danach in einem zeitlich getrennten Arbeitsschritt in Form von mindestens einem Band kontinuierlich auf einem parallel verlaufenden wesentlich breiteren Band aus Teststreifenträgermaterial in einem Durchlaufverfahren befestigt wird, und schließlich das Gesamtband quer zu seiner Längsrichtung in eine Vielzahl von Teststreifen zerschnitten wird. Weiter richtet sich die Erfindung auf einen Teststreifen zur Analyse von Bestandteilen einer Flüssigkeit, insbesondere einer Körperflüssigkeit des Menschen, mit einem länglichen Träger mit einem Vorderende und einem Hinterende, wobei näher bei dem Vorderende mindestens ein die Analyse ermöglichendes Testfeld angebracht ist und näher am Hinterende ein Handhabungsbereich vorgesehen ist, um den Teststreifen mit der Flüssigkeit zu befeuchten und der Auswertung zuzuführen und mit einer maschinenlesbaren Codierung in Form von im wesentlichen quer zum Teststreifen verlaufenden Strichen.

In den letzten Jahren werden analytische Bestimmungen, insbesondere für medizinische Zwecke, in zunehmendem Maße mit Hilfe von Teststreifen durchgeführt. Die Handhabung dieser Teststreifen ist sehr einfach. Urinteststreifen werden im allgemeinen kurz eingetaucht und die Flüssigkeit anschließend einfach abtropfen gelassen. Für Blutuntersuchungen wird üblicherweise ein Blutstropfen aufgegeben und wieder abgewischt, nachdem sich das Testfeld vollgesaugt hat. Es sind aber auch andere Vorgehensweisen möglich. Bei den üblichen Teststreifen spielt sich auf den Testfeldern eine chemische Reaktion zwischen Inhaltsstoffen der Körperflüssigkeit und auf dem Testfeld vorhandenen Reagenzien ab, die zu einer Farbveränderung des Testfeldes führt. Die Farbveränderung wurde in der Anfangszeit der Analyse mit Teststreifen visuell ausgewertet.

Die visuelle Auswertung des Farbumschlages erlaubt nur eine qualitative oder semiquantitative Aussage über die Konzentration des zu analysierenden Stoffes. Wegen der einfachen Handhabung und insbesondere der für kleinere Untersuchungsserien günstigen Kosten der Teststreifen wird jedoch bereits seit längerem eine quantitative Auswertung der Farbveränderung mit Hilfe entsprechender Geräte angestrebt. Üblicherweise verwendet man Reflexionsphotometer, die den Reflexionsgrad der Testfeldoberfläche nach Ablauf der Reaktion bei einer oder mehreren Wellenlängen bestimmen.

Ein schwieriges Problem bei diesen Bemühungen wird dadurch verursacht, daß sich die Testfelder der Teststreifen, die üblicherweise aus mit entsprechenden Reagenzien getränkten und getrockneten Papieren oder Vliesen bestehen, nicht so gut reproduzierbar herstellen lassen, daß sie auch dann mit der gewünschten Genauigkeit ausgewertet werden können, wenn sie aus verschiedenen Herstellungschargen stammen. Es gibt deswegen schon verschiedene Vorschläge, dem Auswertegerät eine für die bestimmte Herstellungscharge typische, also chargenspezifische Information, insbesondere die jeweilige Abhängigkeit der Konzentration der zu analysierenden Substanz von dem jeweiligen Reflexionsgrad, zur Verfügung zu stellen.

Beispielsweise werden für diesen Zweck Wechselskalen verwendet, die den Teststreifenpackungen jeweils beigegeben sind und in das entsprechende Gerät eingelegt werden können, um es auf die jeweilige Charge zu eichen. Die notwendige Auswerteinformation kann auch in maschinenlesbarer Form, beispielsweise als Loch- oder Magnetkarten der Packung beigegeben sein. Diese Verfahrensweise weist jedoch erhebliche Nachteile auf. Insbesondere besteht eine große Verwechslungsgefahr, weil die Wechselskalen oder Lochkarten jeweils bei Anbruch einer neuen Teststreifenpackung von Hand ausgewechselt werden müssen. Wird dies versäumt, so kommt es unvermeidlich zu Fehlmessungen, die insbesondere im medizinischen Bereich schwerwiegende Folgen haben können. Ein Teststreifen mit Strichcode ist bekannt, siehe FR-A-2 258 630 = US-A-3 907 503 = DE-A1-2 502 013.

Der Erfindung liegt daher die Aufgabe zugrunde, die zur chargenspezifischen Auswertung notwendige Informationsmenge (größenordnungsmäßig mindestens etwa 50 bit) auf dem Teststreifen selbst dergestalt unterzubringen, daß sie für das Auswertegerät problemlos maschinell lesbar ist und das Gerät leicht bedient werden kann. Das entsprechende Verfahren soll sich dem bisher üblichen Herstellungsverfahren für Teststreifen anpassen und rationell durchführbar sein.

Diese Aufgabe wird bei einem Verfahren der eingangs näher bezeichneten Art dadurch gelöst, daß auf das Teststreifenträgerband vor dem Zerteilen ein parallel zu den Begrenzungen des Teststreifenträgerbandes verlaufender Strichcode hoher Informationsdichte aufgebracht wird, der eine chargenspezifische Information, zur quantitativen Auswertung der Reaktion enthält, die zuvor durch Untersuchung des Testfeldmaterials einer Charge gewonnen wurde. Der eingangs näher bezeichnete erfindungsgemäße Teststreifen ist dadurch gekennzeichnet, daß die Codierung als Strichcode hoher Informationsdichte ausgebildet ist, der als Speicher chargenspezifische zur quantitativen Auswertung der Reaktion notwendige, durch Untersuchung des Testfeldmaterials einer Charge gewonnene Information enthält.

Dieser zunächst einfach erscheinenden Lösung standen eine Reihe schwerwiegender Probleme entgegen. Zu berücksichtigen ist zunächst, daß die üblichen Teststreifen sehr kleine Abmessungen (ca. 6 mm × 80 mm) haben. Aus verschiedenen im folgenden nach näher erläuterten Gründen ist hiervon nur ein Teil für die chargenspezifische Codierung verfügbar, beispielsweise eine Fläche von ca. 30 mm × 6 mm. Andererseits beträgt die für die chargenspezifische Codierung, insbesondere die Angabe der Eichkurve zwischen Reflexion und Konzentration notwendige Informationsmenge größenordnungsmäßig mindestens ca. 50 bit. Zu diesen sehr beengten Platzverhältnissen kommen erschwerende fertigungstechnische Randbedingungen. Die Teststreifen müssen sehr schnell, zuverlässig und kostengünstig hergestellt werden. Dabei muß sich die Aufbringung der Codierung möglichst an das bewährte eingangs näher bezeichnete Verfahren zur Teststreifenherstellung anpassen. Das Testfeldmaterial darf durch das Aufbringen der Codierung auf die Teststreifen nicht derart beeinflußt werden, daß sich seine zuvor gemessenen Eigenschaften, die ja zu der chargenspezifischen Codierung führen, während des Aufbringvorganges wieder verändern. Aufgrund dieser unüberwindlich erscheinenden Probleme ist offenbar bisher nicht vorgeschlagen worden, einen an sich, beispielsweise zur Kennzeichnung von Lebensmittelpackungen, bekannten Strichcode hinreichend hoher Informationsdichte im Sinne der vorliegenden Erfindung zu verwenden.

Dieses in der Fachwelt offenbar bestehende Vorurteil wird dadurch besonders deutlich, daß in jüngerer Zeit Teststreifen und ein entsprechendes Auswertegerät bekannt geworden sind, die bereits mit einer maschinenlesbaren Codierung in Form von im wesentlichen quer zum Teststreifen verlaufenden Strichen versehen sind. Diese Codierung ist jedoch nur zur Identifikation des jeweils in das Gerät eingelegten Teststreifentypes (also der Art der durchzuführenden Analyse) geeignet, weil, offenbar wegen der oben beschriebenen herstellungstechnischen Probleme, die Informationsdichte sehr gering ist. Die kleinste Breite der Codestriche und der Abstände zwischen ihnen liegt in diesem Fall bei mehr als 1 mm, während für die erfindungsgemäße chargenspezifische Codierung unter den auf den üblichen Teststreifen gegebenen Bedingungen eine Maximalbreite der kleinsten Striche von ca. 100 bis 200 μm notwendig ist. Die Codierung wird bei dem bekannten Gerät mit einem den Teststreifen durchdringenden Lichtstrahl abgelesen. Demgegenüber wird der Strichcode bei der vorliegenden Erfindung bevorzugt mit einem Reflexionsverfahren ausgewertet, da im Rahmen der Erfindung gefunden wurde, daß die Störungen des Lichtstrahls in dem ca. 300 μm starken Teststreifen ein zuverlässiges Lesen eines Strichcode so hoher Informationsdichte im Transmissionsverfahren sehr erschweren.

Die zur Auswertung notwendige chargenspezifische Information kann nach Herstellung des Testfeldmaterials, üblicherweise also des mit Reagenzien getränkten und getrockneten Testfeldpapieres an diesem gewonnen werden. Bevorzugt wird aber erst aus dem Testfeldmaterial und dem Teststreifenträger eine bandförmige Einheit hergestellt, die den fertigen Teststreifen vollkommen entspricht, aber noch nicht in die einzelnen Streifen durchschnitten ist. Von diesem Band können dann sehr einfach durch Ausschneiden entsprechender Probeteststreifen mehrere für die jeweilige Charge typische Exemplare gewonnen werden. Dieses Verfahren hat den Vorteil, daß die Probeteststreifen mit Geräten untersucht werden können, die den zu späteren Analyse verwendeten Geräten sehr ähnlich sind, d. h. die Bestimmung der chargenspezifischen Eigenschaften erfolgt unter besonders realistischen Bedingungen. Außerdem wird durch dieses bevorzugte Verfahren vermieden, daß eine eventuelle Veränderung der Eigenschaften des Testfeldmaterials durch das Aufbringen auf das Teststreifenträgerband zu einer entsprechenden Verfälschung der Meßergebnisse führt.

Der Strichcode kann auf verschiedenerlei Weise auf das Teststreifenträgermaterial aufgebracht werden. Eine mögliche Lösung sieht beispielsweise vor, daß ein Papierstreifen mit dem jeweiligen Code bedruckt und dann parallel zu dem Testfeldband an geeigneter Stelle auf das Teststreifenträgerband aufgeklebt wird. Statt des Papieres läßt sich auch eine geeignete Folie für diesen Zweck verwenden. Derartige Verfahren sind jedoch verhältnismäßig aufwendig. Besonders bevorzugt wird deshalb der Strichcode mit Hilfe einer Zylinderwalze unmittelbar auf das laufende Trägerband aufgebracht, wobei das Trägerband zwischen der Zylinderwalze und einer Andrückeinrichtung hindurchläuft.

Ein derartiges Verfahren ist prinzipiell mit Hilfe einer üblichen Drucktechnik, also in der Form möglich, daß eine flüssige Farbe beispielsweise durch eine mit einem entsprechenden Relief versehene Zylinderwalze auf den Teststreifen aufgebracht und getrocknet wird. Ein derartiges Druckverfahren führt jedoch zu erheblichen Schwierigkeiten, wenn, wie bei der großtechnischen Herstellung von Teststreifen gegebenenfalls notwendig, Codestriche von einer Breite von beispielsweise nur 100 oder 200 μm in sehr engen Abständen sauber auf ein Trägerband von 100 bis 200 m Länge aufgedruckt werden sollen. Man muß dabei bedenken, daß die Codestriche, um sicher lesbar zu sein, möglichst scharfe Ränder haben und sowohl zueinander als auch bezüglich ihrer Lage auf dem Teststreifen exakt positioniert sein müssen. Diese Forderung wird noch dadurch verschärft, daß selbst bei Verwendung der vorliegenden Erfindung, die sonst bei Strichcodierungen üblichen Sicherungscodes aus Platzmangel nur in sehr geringem Umfang verwendet werden können. Deswegen werden an die Qualität der Auftragung der Codestriche besonders hohe Anforderungen gestellt.

Diese Anforderungen werden erfüllt durch eine bevorzugte Ausführungsform der Erfindung, bei der nicht eine flüssige Farbe, sondern eine feste Farbschicht von einer Farbschichtträgerfolie auf das Teststreifenträgerband unter Anwendung von Druck und gegebenenfalls unter Erhitzen übertragen wird. In diesem Fall entfällt das Trocknen und es läßt sich eine außerordentlich präzise Codierung über die gesamte bei der Teststreifenherstellung übliche Länge des Teststreifenträgerbandes aufbringen. Die Farbschicht selbst kann dabei weniger als 1 μm dick sein.

Besonders bevorzugt wird zu diesem Zweck das sogenannte Heißsiegelverfahren. In diesem Fall befindet sich auf der Farbschichtträgerfolie überlicherweise eine Schicht aus Trennlack, darauf die zu übertragende Farbschicht und zu oberst eine Schicht aus Heißsiegelmittel, einem sich in der Hitze verflüssigenden und eine Klebewirkung entfaltenden Polymer. Zur Aufbringung der Farbschicht wird in diesem Fall eine mit einem entsprechenden Relief versehene erhitze Zylinderwalze verwendet, die von hinten gegen die Farbschichtträgerfolie drückt, dadurch das Siegelmittel zum Schmelzen bringt und die Farbschicht mit dem Trennlack auf das Teststreifenträgerband überträgt. Hierfür geeignet Heißsiegelfolien werden beispielsweise von der Firma L. Kurz, Fürth i. Bayern unter den Bezeichnungen Prägefolie « Alufin » oder « Luxor » angeboten.

Zum Aufbringen des Strichcodes eignen sich zahlreiche Materialien. Besonders bevorzugt sind die in fester Form und insbesondere durch Heißsiegeln übertragbaren Kunststoffmaterialien, die Pigmente in einem entsprechenden Bindemittel enthalten. Besonders bevorzugt bestehen die Striche jedoch aus einem Material, das metallische Bestandteile enthält. Derartige Striche sind vor allen Dingen dann sinnvoll, wenn zum Lesen des Strichcode infrarotes Licht verwendet wird. Die üblichen als Teststreifenträgermaterial verwendeten Kunststoffe reflektieren Infrarotlicht nämlich nur relativ schwach, während metallische Codestriche reflektierend wirken. Dadurch ergibt sich ein besonders guter Konstrast, der die Lesbarkeit des Strichcode erhöht. Schließlich sind als Lichtquelle für den Strichcodeleser Infrarot-Leuchtdioden besonders gut geeignet, weil sie bei gegebener Leistungsaufnahme eine besonders hohe Lichtintensität haben.

Die Erfindung wird im folgenden anhand eines in den Figuren dargestellten Ausführungsbeispieles näher erläutert. Es zeigen :

Figur 1 eine schematische perspektivische Ansicht eines erfindungsgemäßen Teststreifens schräg von oben,

Figur 2 einen Teststreifen nach Fig. 1 schräg von unten,

Figur 3 eine stark schematisierte perspektivische Darstellung einer Vorrichtung zum Aufbringen eines Strichcode auf ein Band aus Teststreifenträgermaterial,

Figur 4 eine Zylinderwalze für eine Vorrichtung nach Fig. 3 im Querschnitt und

Figuren 5a und 5b eine Aufsicht auf einen Druckring bzw. einen Zwischenring für eine Zylinderwalze nach Fig. 4.

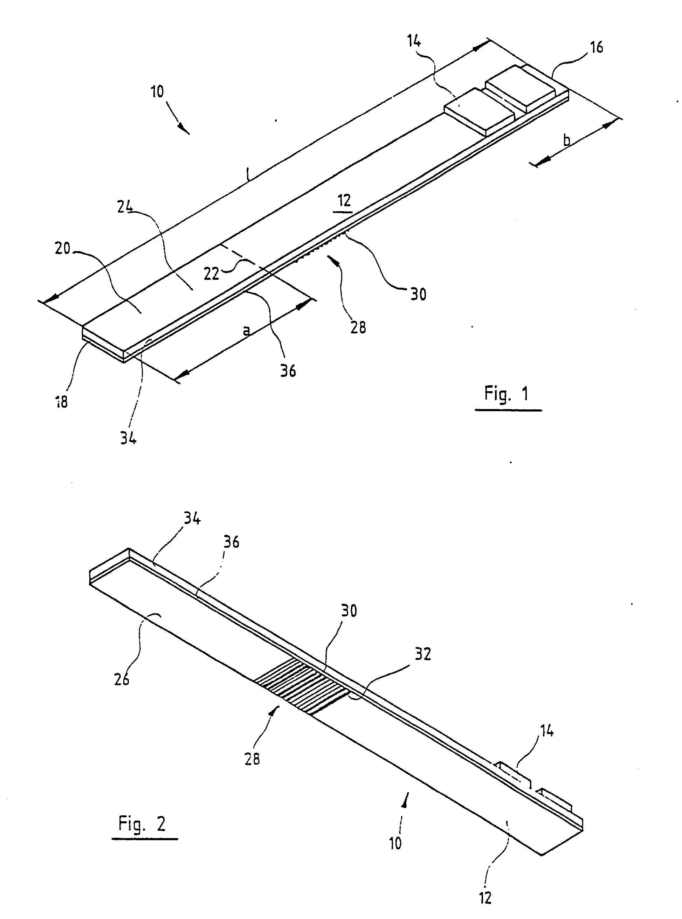

Fig. 1 und Fig. 2 zeigen einen in seiner Gesamtheit mit dem Bezugszeichen 10 versehenen Teststreifen. Der Teststreifen 10 besteht im wesentlichen aus dem Teststreifenträger 12 und einem oder mehreren Testfeldern, wobei in den Figuren ein Teststreifen mit zwei Testfeldern 14 dargestellt ist.

Die Testfelder 14 befinden sich in der Nähe des Vorderendes 16 des in seiner Gesamtheit länglich ausgebildeten Teststreifenträgers 12. Am gegenüberliegenden Ende, das als Hinterende 18 bezeichnet wird, befindet sich ein Handhabungsbereich 20, der durch die gestrichelte Linie 22 begrenzt wird.

Die Testfelder 14 befinden sich auf der Oberseite 24 des Teststreifenträgers, die in Fig. 1 zu erkennen ist. Fig. 2 zeigt die Unterseite 26 deutlicher. Man erkennt einen in seiner Gesamtheit mit dem Bezugszeichen 28 versehenen Strichcode, der aus einzelnen verschieden breiten, im wesentlichen quer zur Längsrichtung des Teststreifens 10 verlaufenden Codestrichen 30 besteht.

Bevorzugt wird sowohl die Breite der Codestriche als auch ihr Abstand zueinander für die gewünschte chargenspezifische Codierung verwendet. In einer praktischen Ausführungsform sind die Codestriche entweder 200 μm oder 400 μm breit, wobei die schmalen Striche eine logische « 0 » und die breiten Codestriche eine logische « 1 » repräsentieren. Entsprechend werden zwei verschiedene Abstände zwischen Codestrichen verwendet. Außerdem weist der Code einen Startbalken 32 auf. Der Stratbalken 32 oder ein anderer geeigneter Teil der Codierung kann auch zugleich zur Kontrolle der richtigen Positionierung des Teststreifens 10 in dem entsprechenden Auswertegerät verwendet werden. Eine Codierung der hier beschriebenen Art, bei der sowohl die Breite der Codestriche 30 als auch die Breite der Abstände zwischen den Codestrichen zur Codierung verwendet wird, zeichnet sich durch eine besonders hohe Informationsdichte aus, erfordet aber andererseits eine besonders präzise Herstellung des Strichcode, da sonst bereits verhältnismäßig geringe Fehler der Bedruckung zu einer Fehlablesung führen können.

Diese hohe Informationsdichte ist notwendig, um die für die chargenspezifische Auswertung notwendige Informationsmenge von mindestens etwa 50 bit auf dem Teststreifen unterbringen zu können. Die üblichen Teststreifen haben eine Gesamtlänge 1 (Fig. 1) von ca. 70 bis 80 mm. Diese Länge hat sich praktisch bewährt und stellt einen guten Kompromiß zwischen optimaler Handhabung, relativ geringen Herstellungskosten und ausreichender Biegefestigkeit des Teststreifens 10 dar. Diese Länge soll deshalb möglichst nicht überschritten werden.

Von der Gesamtlänge 1 wird eine Teillänge b von den Testfeldern beansprucht. Dieser Bereich sollte nach Möglichkeit aus den folgenden zwei Gründen nicht für das Aufbringen des Strichcode verwendet werden. Einerseits wäre es relativ schwierig, einen auf der Unterseite 26 des Teststreifenträgers 12 im Bereich der Testfelder angebrachten Strichcode 28 in einem entsprechenden Teststreifenauswertegerät abzulesen, da eine vor entsprechend präzise Führung des Teststreifens 10 für das Lesen des Strichcode 28 notwendig ist, die im Bereich der Testfelder 14 nur mit Schwierigkeiten zu realisieren wäre. Andererseits wäre auch die Herstellung eines Teststreifens 10 mit einem unter den Testfeldern 14 vorhandenen Strichcode problematisch. Wie bereits weiter oben erwähnt, sollten die Testfelder 14 nach Möglichkeit auf den Teststreifenträger 12 aufgebracht sein, bevor die Eigenschaften der Testfelder 14 vermessen werden, um die zur Auswertung nötige Konzentrations-Reflexions-Abhängigkeit zu bestimmen, die dann in Form des Strichcode 28 auf den Teststreifen 10 aufgebracht wird. Beim Aufbringen des Strichcode 28 ist aber, wie im folgenden anhand des erfindungsgemäßen Verfahrens noch näher beschrieben wird, die Anwendung von Druck und gegebenenfalls erhöhter Temperatur notwendig. Wäre der Strichcode 28 im Bereich der Testfelder 14 angebracht, so bestünde die Gefahr, daß sich die Eigenschaften der Testfelder 14 beim Aufbringen des Strichcode 28 wieder verändern und somit die in dem Strichcode 28 enthaltene Information nicht mehr den tatsächlichen Verhältnissen entspricht.

Auch der Handhabungsbereich 20 (Länge a in Fig. 1) sollte nach Möglichkeit nicht für den Strichcode 28 verwendet werden. Bei relativ einfachen Auswertegeräten beispielsweise sollte der Teststreifen von Hand in eine entsprechende Aufnahmeöffnung eingeschoben und wieder entnommen werden können. In diesem Fall muß notwendigerweise ein Teil des Teststreifens 10 aus dem Gerät herausragen. Dieser Teil ist dann für den Strichcode keinesfalls nutzbar. Diese Problematik besteht nicht bei Auswertegeräten, bei denen die Teststreifen mechanisch in das Gerät eingezogen werden. Auch in diesem Fall ist es jedoch günstiger, den Handhabungsbereich 20 nicht für den Strichcode zu benutzen.

Man erkennt aus den vorstehenden Ausführungen, daß die für den Strichcode 28 zur Verfügung stehende Teillänge s = 1 — (a + b) vom Einzelfall abhängt. Geht man von dem praktisch realistischen Beispiel aus, daß der Anfaßbereich eine Länge von a = 30 mm hat und der Bereich der Testfelder eine Länge von b = 15 mm (einschließlich eines geringen Sicherheitsabstandes), so ergibt sich bei einer Teststreifenlänge von 1 = 75 mm, daß nur ca. 30 mm für den Strichcode zur Verfügung steht.

Geht man weiterhin von einem notwendigen Informationsgehalt des Strichcode 28 von ca. 60 bit aus, so ergibt sich eine Informationsdichte von 20 bit/cm. Eine derartig hohe Informationsdichte ist von konventionellen Strichcodes für andere Anwendungsfälle zwar durchaus bekannt. Bei der Herstellung von Teststreifen besteht jedoch die bereits weiter oben beschriebene besondere Problematik, aufgrund derer die Fachwelt offenbar der Meinung war, daß sich ein Strichcode nicht für die gewünschte chargenspezifische Information auf Teststreifen verwenden lasse. Diese Problematik wurde durch das im folgende näher beschriebene erfindungsgemäße Verfahren überwunden.

Für das hier beschriebene bevorzugte Verfahren ist es zunächst wesentlich, daß der Teststreifenträger 12 aus zwei miteinander verbundenen, bevorzugt coextrudierten Schichten aufgebaut ist. Die obere Schicht 34 ist bevorzugt ca. 300 μm dick und besteht aus einem Kunststoff, der die Notwendige Steifigkeit aufweist, bevorzugt Polystyrol. Die der Unterseite des Teststreifens 10 zugewandte Schicht ist sehr viel dünner als die obere Schicht 34, bevorzugt ca. 60 μm stark und besteht aus einem Material, auf dem sich die Codestriche in dem in folgenden beschriebenen Heißsiegelverfahren besonders gut aufbringen lassen. Dieses Material sollte einen relativ niedrigen Erweichungspunkt haben, so daß schon bei relativ niedrigen Temperaturen und relativ geringen Drücken eine zuverlässige Verbindung mit der den Strichcode 28 bildenden Farbschicht erreicht wird, ohne daß sich an der Unterseite 26 des Teststreifensträgers 12 zu starke Deformationen ergeben. Besonders bewährt hat sich zu diesem Zweck Polyäthylen oder Äthylenvinylacetat. Ebenfalls geeignet sind Polyamid und Acrylnitril-Butadien-Styrol.

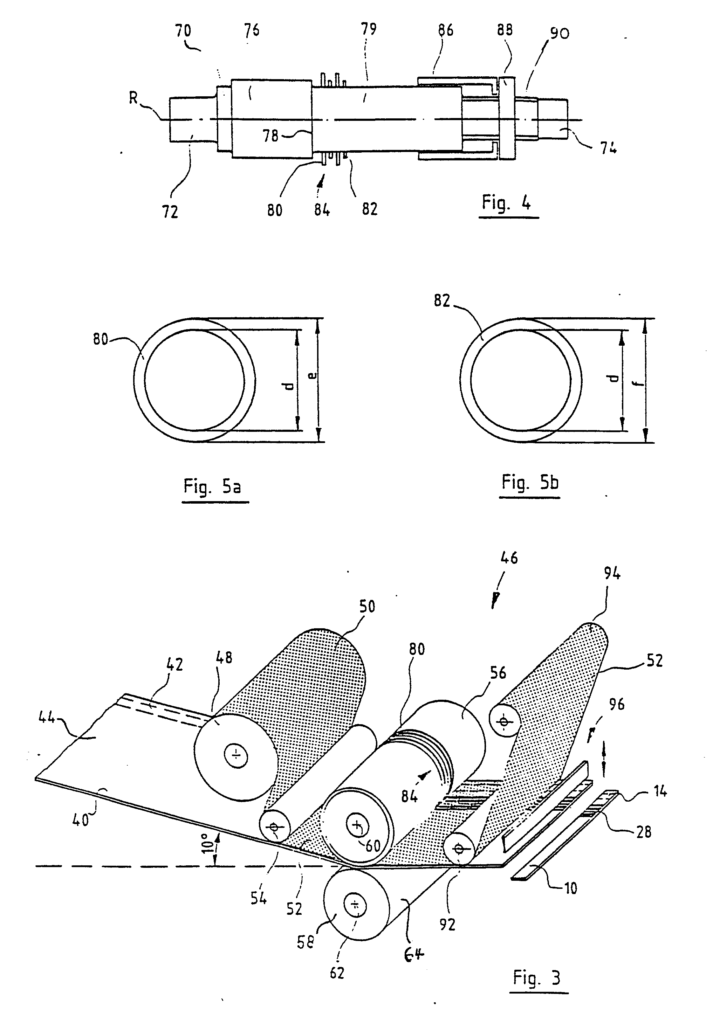

Ein bevorzugtes Verfahren gemäß der vorliegenden Erfindung wird im folgenden anhand der Fig. 3 beschrieben, wobei die Fig. 3 lediglich das Aufbringen des Barcodes 28 in dem bevorzugten Heißsiegelverfahren zeigt. Zuvor ist das Teststreifenträgerband 40 bereits mit dem Testfeldband 42 verbunden worden.

Üblicherweise werden Teststreifen dergestalt hergestellt, daß man auf einem langen Band (z. B. 100 bis 200 m lang) aus Teststreifenträgermaterial, dessen Breite einem ganzzahligen Vielfachen der späteren Teststreifenlänge entspricht, an den entsprechenden Stellen über die ganze Länge ein oder mehrere sehr viel schmalere Bänder 42 aus Testfeldmaterial anbringt. Die Breite der Testfeldbänder 42 entspricht deren späterer Länge auf dem fertigen Teststreifen. Dieser Verfahrensschritt wird üblicherweise in einem kontinuierlichen Vorgang durchgeführt, wobei das Teststreifenträgerband 40 und das Testfeldband 42 von entsprechenden Rollen abgezogen werden und gemeinsam eine Einrichtung durchlaufen, durch die die Verbindung zwischen beiden Bestandteilen hergestellt wird. Danach wird (gegebenenfalls nach einer Trocknungs- oder Aushärtephase) das aus Teststreifenträgerband und den aufgebrachten Testfeldbändern bestehende Gesamtband, das im folgenden auch als Teststreifenband 44 bezeichnet wird, in einer Richtung quer zu seiner Längserstreckung in Stücke zerschnitten, deren Breite der Breite der fertigen Teststreifen entspricht. Wenn das ursprüngliche Teststreifenträgerband 40 in seiner Breite der Länge von mehreren Teststreifen entsprach, so wird das Gesamtband vor dem Zerschneiden in Querrichtung noch in Längsrichtung in mehrere Teilbänder aufgespalten, wobei die Breite jedes dieser Teilbänder der Länge eines Teststreifens entspricht. Die Verbindung zwischen Teststreifenträgerband und Testfeldband kann in einer Vielzahl verschiedener bekannter Weisen, beispielsweise durch geeignete Klebeverfahren, erfolgen, wobei die verwendeten Materialien zu berücksichtigen sind. Dieser Verfahrensschritt ist nicht Bestandteil der vorliegenden Erfindung und wohlbekannt, so daß hier nicht weiter darauf eingegangen wird.

Für die vorliegende Erfindung wesentlich ist, daß bei der Verbindung von Testfeldband 42 und Teststreifenträgerband 40 sich die Eigenschaften des Testfeldmaterials bezüglich der durchzuführenden Analyse möglicherweise noch verändern können. Dies ist einer der Gründe, warum es besonders bevorzugt ist, wenn zunächst die Verbindung zwischen Testfeldband 42 und Teststreifenträgerband 40 hergestellt und das daraus resultierende Gesamtband 44, beispielsweise in Form einer Rolle, gegebenenfalls zwischengelagert wird, bevor der Barcode für die chargenspezifische Auswertung ermittelt und aufgebracht wird. Aus der Rolle mit dem Gesamtband lassen sich leicht mehrere Teststreifen schneiden, die als Proben für die Ausmessung der Reflexions-Konzentrations-Abhängigkeit der jeweiligen Charge verwendet werden können. Vorteilhafterweise verwendet man zur Auswertung Geräte, die im wesentlichen den später auch für die eigentliche Analyse angewendeten Geräten entsprechen, um möglichst realistische Bedingungen zu erreichen.

Hat man die gewünschte Reflexions-Konzentrations-Kurve ermittelt, so läßt sie sich mit Hilfe eines geeigneten mathematischen Verfahrens, das nicht Gegenstand der vorliegenden Erfindung ist, in Form einer mathematischen Funktion darstellen, die nach einem bekannten Verfahren in einem maschinenlesbaren Code übertragen werden kann. Der Code kann außerdem weitere Informationen, beispielsweise über den mit dem jeweiligen Teststreifen durchzuführenden Test, enthalten.

Fig. 3 zeigt, wie dieser Code bevorzugt auf das aus Teststreifenträgerband 40 und Testfeldband 42 bestehende Gesamtband 44 aufgebracht wird. Das Gesamtband 44 läuft unter einem Winkel von ca. 10° zur Horizontalen von einer nicht dargestellten Rolle in die Codeaufbringungseinrichtung ein, die in ihrer Gesamtheit mit dem Bezugszeichen 46 versehen ist. Dabei befindet sich das Testfeldband 42 auf der in der Figur nicht zu erkennenden Unterseite des Gesamtbandes 44 und ist deshalb gestrichelt dargestellt.

Oberhalb des einlaufenden Bandes 44 befindet sich eine Rolle 48 mit einer Heißsiegelfolie 50. Die Heißsiegelfolie besteht im wesentlichen aus einer etwa 12 μm starken Polyesterfolie, auf der sich ein Schutz- und Trennlack, die für die Codestriche notwendige Farbschicht und eine Siegelschicht befinden. Die Heißsiegelfolie 50 ist auf der Rolle 48 dergestalt aufgewickelt, daß die den Farbschichtträger bildende Polyesterfolie 52 auf der Rolle 48 außen ist.

Von der Rolle 48 gelangt die Heißsiegelfolie über eine Umlenkrolle 54 zwischen eine Heißsiegelwalze 56 und eine Andruckwalze 58, die sich um Achsen 60 bzw. 62 drehen. Beide Achsen verlaufen parallel zu dem Teststreifenband 44. Die Heißsiegelwalze 56 ist mit Hilfe einer nicht dargestellten innenliegenden Heizpatrone beheizt. Sie besteht, wie im folgenden noch eingehender beschrieben wird, aus einer Edelstahl-Hohlwelle und aufschiebbaren Druckringen mit zugehörigen Befestigungseinrichtungen. Die Andruckwalze 58 ist aus Stahl hergestellt, wobei ihre Oberfläche 64 blankpoliert ist.

Der Einlaufwinkel von 10° ist für die weiter hinten angegebenen Verfahrensbedingungen besonders bevorzugt. Allgemein ist es vorteilhaft, wenn das Testband 44 die Heißsiegelwalze 56 in geringem Ausmaß umläuft, d. h. der Winkel zwischen Einlaufrichtung und Auslaufrichtung 5 bis 20° beträgt.

Fig. 4 zeigt einen Querschnitt durch eine für das erfindungsgemäße Verfahren bevorzugt verwendete Zylinderwalze. Nicht dargestellt ist die zuvor erwähnte, im Inneren des Walzenkörpers 70 angeordnete Heizpatrone. Der Walzenkörper 70 ist bevorzugt einstückig hergestellt und hat an seinen beiden Enden Lagerachsen 72 und 74, die in entsprechenden Lagern der Maschine laufen können. Die Rotationsachse ist strichpunktiert gezeichnet und mit dem Bezugszeichen R versehen. Benachbart zu der in der Figur linken Lagerachse 72 hat der Walzenkörper 70 eine Verdickung 76, die über eine zur Rotationsachse R senkrechte Kante 78 in die Ringaufnahme 79 übergeht.

Auf der Ringaufnahme 79 sitzen in einer dem gewünschten Strichcode entsprechenden Folge Druckringe 80 und Zwischenringe 82, die insgesamt ein Druckrelief 84 bilden. Sie werden je nach dem gewünschten Strichcode von der in der Figur rechten Seite auf die Ringaufnahme 79 nacheinander aufgeschoben. Bei dem weiter oben erwähnten Codebeispiel werden jeweils Druckringe und Zwischenringe von 200 μm und 400 μm Stärke verwendet. Das erfindungsgemäße Verfahren hat sich aber auch für Codierungen bewährt, bei denen nur 100 μm breite Striche (und demzufolge Druckringe 80) eingesetzt werden.

Die Gesamtheit der Druckringe 80 und Zwischenringe 82 wird durch eine Anpreßmuffe 86 mit Hilfe einer entsprechenden Mutter 88, die auf einem Gewinde 90 aufschraubbar ist, gegen die Kante 78 gepreßt und dadurch arretiert.

Selbstverständlich kann die zum Aufdrucken verwendete Zylinderwalze auch in anderer Weise konstruiert sein. Die hier beschriebene Vorrichtung ist jedoch deshalb besonders bevorzugt, weil sie eine schnelle und einfache Möglichkeit darstellt, den für die jeweilige Charge an Teststreifenband 44 gewünschten Strichcode 28 in die Form eines entsprechenden Druckreliefs 84 zu bringen.

Die Durchmesserunterschiede zwischen den Druckringen 80 und den Zwischenringen 82 sind in Fig. 4 stark übertrieben dargestellt. Die entsprechenden Dimensionen erkennt man besser aus den Fig. 5a und 5b. Der Innendurchmesser beider Ringtypen d entspricht dem Außendurchmesser der Ringaufnahme 79 und beträgt in einem bevorzugten Beispiel 48 mm. Der Außendurchmesser e des Druckringes 80 liegt in diesem Beispiel bei 60 mm, während der Außendurchmesser f des Zwischenringes 82 59 mm beträgt. Der Radienunterschied beider Ringtypen beträgt also in diesem Fall nur 0,5 mm. Eine derartige Konstruktion hat sich bei dem besonders bevorzugten Heißsiegelverfahren bewährt, weil das Druckrelief 84 einerseits ausreichend tief ist, um eine saubere Bedruckung zu erreichen und andererseits auch sehr dünne Druckringe 80 noch die notwendige Steifigkeit und Stabilität haben.

Wie aus den Fig. 4 und 3 zu ersehen ist, nimmt der von dem Druckrelief 84 eingenommene Raum auf der Heißsiegelwalze 56 nur einen verhältnismäßig kleinen Teil von deren Breite ein. Entsprechend wird die Rückseite des Teststreifenbandes 44 aus den weiter oben beschriebenen Gründen nur in dem relativ schmalen Bereich zwischen den Testfeldern 14 bzw. 42 und dem Handhabungsbereich 20 bedruckt.

Durch den angewendeten Druck und die Erhitzung mit Hilfe der Heißsiegelwalze 56 wird die Farbschicht auf dem Farbschichtträger 52 an der Stelle und jeweils in der Breite auf das Teststreifenband 44 übertragen, wo sich innerhalb des Druckreliefs 84 ein Druckring 80 befindet. Diese Übertragung einer festen Farbschicht von einem Farbschichtträger ist für die Codierung eines Teststreifens unter den erwähnten schwierigen Bedingungen besonders geeignet, weil die Codestriche präzise und sauber übertragen werden und kein anschließender Trocknungsvorgang notwendig ist.

Die Codestriche werden durch den auf der Heißsiegelfolie 50 befindlichen Schmelzkleber befestigt und sind mit dem sich ebenfalls von dem Farbschichtträger 52 ablösenden Schutz- und Trennlack bedeckt.

Die übrig gebliebene Farbschichtträgerfolie 52 wird über eine zweite Umlenkrolle 92 auf eine Aufnahmerolle 94 aufgewickelt. Das Teststreifenband 44 gelangt zu einer nur schematisch dargestellten Schneideeinrichtung 96, wo es quer zu seiner Transportrichtung in die einzelnen Teststreifen 10 zerschnitten wird.

Bei der hier dargestellten bevorzugten Version des erfindungsgemäßen Verfahrens werden die Codestriche 28 als Farbschicht, also in Form einer außerordentlich dünnen Folie unmittelbar auf den Träger übertragen. Obwohl diese Version wegen ihrer Einfachheit und der Qualität des erzielten Produktes besonders bevorzugt ist, kann es in anderen Anwendungsfällen zweckmäßig sein, den Strichcode zunächst auf ein entsprechendes Bandmaterial aufzudrucken und dann parallel zu dem Testfeldband 42 auf dem Teststreifenträgerband 40 zu befestigen. Besonders bevorzugt eignet sich hierfür eine Kunststoffolie, die zunächst mit dem Strichcode bedruckt und anschließend mit dem Trägerband 40 verbunden wird.

Bei dem bereits mehrfach erwähnten Beispiel, bei dem der Strichcode aus ca. 60 bit mit 200 μm und 40 μm breiten Strichen und Abständen besteht, hat es sich bewährt, mit einer Oberflächentemperatur der Druckringe 80 von ca. 130 °C und einer Vorschubgeschwindigkeit von ca. 4 m/min zu arbeiten. Die Andruckkraft zwischen Heißsiegelwalze 56 und Andruckwalze 58 betrug dabei ca. 400 Newton (N).

**Patentansprüche**

1. Verfahren zum Herstellen eines Analysetestreifens mit einem länglichen Träger, auf dem mindestens ein Testfeld befestigt ist, bei dem das Testfeldmaterial chargenweise in einer für eine Vielzahl von Teststreifen ausreichenden Menge hergestellt und danach in einem zeitlich getrennten Arbeitsschritt in Form von mindestens einem Band auf einem parallel verlaufenden wesentlich breiteren Band aus Teststreifenträgermaterial in einem Durchlaufverfahren befestigt wird und schließlich das Gesamtband quer zu seiner Längsrichtung in eine Vielzahl von Teststreifen zerteilt wird, dadurch gekennzeichnet, daß auf das Teststreifenträgerband vor dem Zerteilen ein parallel zu den Begrenzungen des Teststreifenträgerbandes verlaufender Strichcode hoher Informationsdichte aufgebracht wird, der eine chargenspezifische Information zur quantitativen Auswertung der Reaktion enthält, die zuvor durch Untersuchung des Testfeldmaterials einer Charge gewonnen wurde.

2. Verfahren nach Anspruch 1, dadurch gekennzeichnet, daß die zur Auswertung notwendige chargenspezifische Information nach dem Befestigen des Testfeldmaterials auf dem Teststreifenträger gewonnen wird.

3. Verfahren nach einem der Ansprüche 1 oder 2, dadurch gekennzeichnet, daß der Strichcode mit Hilfe einer Zylinderwalze unmittelbar auf das laufende Trägerband aufgebracht wird, wobei das Trägerband zwischen der Zylinderwalze und einer Andruckeinrichtung hindurchläuft und die Zylinderwalze um eine Achse parallel zur Bandfläche und quer zur Transportrichtung des Bandes rotiert.

4. Verfahren nach Anspruch 3, dadurch gekennzeichnet, daß die Zylinderwalze vor dem Aufbringen des Strichcode für eine Charge Testfeldmaterial aus einer Mehrzahl von verschieden breiten Druckringen und Zwischenringen zusammengesetzt wird, die konzentrisch auf einer gemeinsamen Achse angebracht werden und deren Breite und Aufeinanderfolge dem jeweils gewünschten Strichcode entspricht.

5. Verfahren nach einem der Ansprüche 3 oder 4, dadurch gekennzeichnet, daß die Striche des Strichcode als Farbschicht von einem mit dem Trägerband zwischen der Zylinderwalze und der Andrückeinrichtung hindurchlaufenden Farbschichtträger übertragen werden.

6. Verfahren nach Anspruch 5, dadurch gekennzeichnet, daß der Farbschichtträger eine Kunststoffolie ist, die eine Schicht aus Trennlack, die Farbschicht und eine Schicht aus Heißsiegelmittel trägt und die Zylinderwalze beheizt ist, so daß die Farbschicht durch Hitze und Druck der Zylinderwalze auf den Teststreifenträger übertragen wird.

7. Verfahren nach einem der Ansprüche 1 bis 6, dadurch gekennzeichnet, daß der Strichcode auf der von den Testfeldern abgewandten Seite des Teststreifens und entfernt von den Testfeldern aufgebracht wird.

8. Teststreifen (10) zur Analyse von Bestandteilen einer Flüssigkeit, insbesondere einer Körperflüssigkeit des Menschen, mit einem länglichen Träger (12), an dem mindestens ein die Analyse ermöglichendes Testfeld (14) angebracht ist, mit einem Handhabungsbereich (20), um den Teststreifen (10) mit der Flüssigkeit zu befeuchten und der Auswertung zuzuführen und mit einer maschinenlesbaren Codierung in Form von im wesentlichen quer zum Teststreifen verlaufenden Strichen, dadurch gekennzeichnet, daß die Codierung als Strichcode (28) hoher Informationsdichte ausgebildet ist, der als Speicher chargenspezifische zur quantitativen Auswertung der Reaktion notwendige, durch Untersuchung des Testfeldmaterials einer Charge gewonnene Information enthält.

9. Teststreifen nach Anspruch 1, dadurch gekennzeichnet, daß der Strichcode (28) als nachträglich mit dem Träger verbundenes Bandmaterial ausgebildet ist.

10. Teststreifen nach einem der Ansprüche 8 oder 9, dadurch gekennzeichnet, daß die Striche (30) des Strichcode im wesentlichen aus einem mit dem Träger (12) nachträglich verbundenen Folienmaterial bestehen und unmittelbar auf dem Träger (12) angebracht sind.

11. Teststreifen nach Anspruch 10, dadurch gekennzeichnet, daß das Folienmaterial eine von einem Farbschichtträger (52) abgelöste durch Heißsiegeln mit dem Trägermaterial (12) verklebte Farbschicht ist.

12. Teststreifen nach einem der Ansprüche 8 bis 11, dadurch gekennzeichnet, daß der Strichcode (28) im infraroten Licht lesbar ist, wobei die Striche (30) so ausgebildet sind, daß sie das Infrarotlicht reflektieren, während der Träger (12) das Infrarotlicht relativ schwach reflektiert.

13. Teststreifen nach einem der Ansprüche 8 bis 12, dadurch gekennzeichnet, daß die Striche (30) des Strichcodes (28) metallische Bestandteile enthalten und der Träger (12) aus Kunststoff hergestellt ist.

14. Teststreifen nach einem der Ansprüche 8 bis 13, dadurch gekennzeichnet, daß der Handhabungsbereich (20) in der Nähe des einen Endes (18) und das mindestens eine Testfeld (14) in der Nähe des anderen Endes (16) des Trägers (12) angeordnet ist und der Strichcode in Längsrichtung des Trägers (12) zwischen dem Handhabungsbereich (20) und dem diesem nächstliegenden Testfeld (14) auf der dem Testfeld (14) gegenüberliegenden Fläche des Teststreifens (10) angebracht ist.

15. Teststreifen nach einem der Ansprüche 8 bis 14, dadurch gekennzeichnet, daß der Strichcode mindestens 30 bit, bevorzugt 50 bit, Information enthält, die Strichbreite der schmalsten Striche nicht größer als 400 μm, bevorzugt 200 μm, ist und im wesentlichen zwei Strichbreiten verwendet werden.

**Claims**

1. Process for the production of an analysis test strip with a longitudinal carrier upon which is fixed at least one test field, in which the test field material is produced batchwise in an amount sufficient for a plurality of test strips and thereafter, in a chronologically separate working step, is fixed in the form of at least one band on to a parallel-running substantially wider band of test strip carrier material in a continuous process and finally the whole band is cut up transversely to its longitudinal direction into a plurality of test strips, characterised in that on to the test strip carrier band, before the cutting up, there is applied a bar code, running parallel to the boundaries of the test strip carrier band, of high information density which contains a batch-specific information for the quantitative evaluation of the reaction which has previously been obtained by investigation of the test field material of a batch.

2. Process according to claim 1, characterised in that the batch-specific information necessary for the evaluation is obtained after the fixing of the test field material on to the test strip carrier.

3. Process according to one of claims 1 or 2, characterised in that the bar code is applied, with the help of a cylindrical roller, directly on to the running carrier band, whereby the carrier band runs through between the cylindrical roller and a pressure device and the cylindrical roller rotates about an axis parallel to the band surface and transversely to the transport direction of the band.

4. Process according to claim 3, characterised in that the cylindrical roller, before the application of the bar code for a batch of test field material, is made up of a plurality of pressure rings and intermediate rings of different breadth, which are applied concentrically to a common axis and the breadth and order of succession of which corresponds to the particular desired bar code.

5. Process according to one of claims 3 or 4, characterised in that the bars of the bar code are transferred as dye layer from a dye layer carrier running through with the carrier band between the cylindrical roller and the pressure device.

6. Process according to claim 5, characterised in that the dye layer carrier is a synthetic resin foil which carries a layer of separating lacquer, a dye layer and a layer of heat seal agent and the cylindrical roller is heated so that the dye layer is transferred to the test strip carrier by heat and pressure of the cylindrical roller.

7. Process according to one of claims 1 to 6, characterised in that the bar code is applied to the side of the test strip remote from the test fields and spaced away from the test fields.

8. Test strips (10) for the analysis of components of a liquid, especially of a human body fluid, with a longitudinal carrier (12) to which is applied at least one test field (14) making possible an analysis, with a handling region (20) in order to be able to moisten the test strip (10) with the liquid to be analysed and to carry out the evaluation and a mechanically-readable coding in the form of bars running substantially transversely to the test strip, characterised in that the coding is constructed as bar code (28) of high information density which contains, as a store, batch-specific information necessary for the quantitative evaluation of the reaction obtained by investigation of the test field material of a batch.

9. Test strip according to claim 8, characterised in that the bar code (28) is formed as a band material subsequently bonded with the carrier.

10. Test strip according to one of claims 8 or 9, characterised in that the bars (30) of the bar code consist essentially of a foil material subsequently bonded with the carrier (12) and applied directly to the carrier (12).

11. Test strip according to claim 10, characterised in that the foil material is a dye layer removed from a dye layer carrier (52) and stuck with the carrier material (12) by heat sealing.

12. Test strip according to one of claims 8 to 11, characterised in that the bar code (28) is readable in infra-red light, whereby the bars are so constructed that they reflect the infra-red light, whereas the carrier (12) reflects the infra-red light relatively weakly.

13. Test strips according to one of claims 8 to 12, characterised in that the bars (30) of the bar code (28) contain metallic components and the carrier (12) is made of synthetic resin.

14. Test strip according to one of claims 8 to 13, characterised in that the handling region (20) is arranged near one end (18) and the at least one test field (14) near the other end (16) of the carrier (12) and the bar code is applied in the longitudinal direction of the carrier (12) between the handling region (20) and the test field (14) lying closest thereto on the surface of the test strip (10) lying opposite to the test field (14).

15. Test strip according to one of claims 8 to 14, characterised in that the bar code contains at least 30 bit, preferably 50 bit, of information, the bar width of the narrowest bar is not greater than 400 µm, preferably 200 µm, and essentially two bar widths are used.

**Revendications**

1. Procédé de fabrication d'une bande d'essai pour analyse comportant un support de forme allongée sur lequel est fixé au moins un champ d'essai, dans lequel le matériau qui constitue le champ d'essai est fabriqué par charges en une quantité suffisante pour plusieurs bandes d'essai et fixé ensuite, dans une opération chronologiquement séparée, sous la forme d'une bande au moins, suivant un procédé continu, sur une bande parallèle, nettement plus large, en matériau constituant le support de bande d'essai, la bande complète étant finalement subdivisée, en travers de sa direction longitudinale, en une pluralité de bandes d'essai, caractérisé en ce qu'avant la subdivision, on réalise sur la bande qui constitue le support de la bande d'essai un code de barres, parallèle aux limites de la bande constituant le support de la bande d'essai, qui contient une information, relative aux charges et permettant l'interprétation quantitative de la réaction, qui a été obtenue auparavant en étudiant le matériau constituant le champ d'essai d'une charge.

2. Procédé selon la revendication 1, caractérisé en ce que l'information relative aux charges nécessaire à l'interprétation est obtenue après la fixation du matériau constituant le champ d'essai sur le support de bande d'essai.

3. Procédé selon l'une des revendications 1 ou 2, caractérisé en ce que le code de barres est réalisé directement sur la bande de support mobile au moyen d'un rouleau cylindrique, la bande support passant entre le rouleau cylindrique et une installation d'impression, et le rouleau cylindrique tournant autour d'un axe parallèle à la surface de la bande et orienté transversalement par rapport à la direction de transport de la bande.

4. Procédé selon la revendication 3, caractérisé en ce que le rouleau cylindrique pour la réalisation du code de barres pour une charge de matériau constituant le champ d'essai est formé d'une pluralité de bagues de compression et de bagues intermédiaires de différentes épaisseurs, qui sont montées ensemble sur un axe commun et dont la largeur et la succession correspondent au code de barres voulu.

5. Procédé selon l'une des revendications 3 ou 4, caractérisé en ce que les barres du code de barres sont transférées sous la forme d'une couche de couleur par un support de couche de couleur qui passe avec la bande support entre le rouleau cylindrique et l'installation d'impression.

6. Procédé selon la revendication 5, caractérisé en ce que le support de couche de couleur est une feuille mince en matériau plastique qui porte une couche de vernis de séparation, la couche de couleur et une couche d'un produit à cacheter à chaud, et en ce que le rouleau cylindrique est chauffé, de sorte que la couche de couleur est transférée par la chaleur et la pression du rouleau cylindrique sur le support de bande d'essai.

7. Procédé selon l'une des revendications 1 à 6, caractérisé en ce que le code de barres est réalisé sur le côté de la bande d'essai qui est du côté opposé aux champs d'essai et éloigné des champs d'essai.

8. Bande d'essai (10) pour l'analyse des constituants d'un liquide, notamment d'un liquide organique provenant du corps humain, comportant un support (12) de forme allongée, sur lequel est placé au moins un champ d'essai (14) permettant l'analyse, et une zoné de manipulation (20), ce qui permet d'humidifier la bande d'essai (10) par le liquide et d'interpréter les résultats obtenus au moyen d'un code lisible par une machine constituée par des barres orientées transversalement par rapport à la bande d'essai, caractérisée en ce que le code est un code de barres (28) de grande densité d'information qui constitue une mémoire contenant l'information correspondant aux charges, qui a été obtenue par l'examen du matériau d'une charge constituant le champ d'essai et permet l'interprétation quantitative de la réaction.

9. Bande d'essai selon la revendication 1, caractérisée en ce que le code de barres (28) est un matériau en bande, relié après coup au support.

10. Bande d'essai selon l'une des revendications 8 ou 9, caractérisé en ce que les barres (30) du code de barres sont constituées essentiellement par une feuille mince reliée après coup au support (12) et sont appliquées directement sur le support (12).

11. Bande d'essai selon la revendication 10, caractérisée en ce que la feuille mince est une couche de couleur détachée d'un suppport (52) de couche de couleur par cachetage à chaud et collée au matériau support (12).

12. Bande d'essai selon l'une des revendications 8 à 11, caractérisée en ce que le code de barres (28) est lisible en lumière infrarouge, les barres (30) ayant une forme telle qu'elles réfléchissent la lumière infrarouge, tandis que le support (12) ne réfléchit que faiblement la lumière infrarouge.

13. Bande d'essai selon l'une des revendications 8 à 12, caractérisée en ce que les barres (30) du code de barres (28) contiennent des constituants métalliques, et en ce que le support (12) est en matière plastique.

14. Bande d'essai selon l'une des revendications 8 à 13, caractérisée en ce que la zone réservée à la manipulation (20) se trouve à proximité de l'une des extrémités (18) du support, en ce qu'au moins un champ d'essai (14) est situé à proximité de l'autre extrémité (16) du support (12), et en ce que le code de barres est appliqué dans le sens de la longueur du support (12), entre la zone de manipulation (20) et le champ d'essai (14) qui est le plus près de cette zone, et sur la surface de la bande d'essai (10) qui est en face du champ d'essai (14).

15. Bande d'essai selon l'une des revendications 8 à 14, caractérisée en ce que le code de barres comprend une information à 30 bits, de préférence une information à 50 bits, en ce que la largeur des barres les plus minces ne dépasse pas 400 μm, de préférence 200 μm, et en ce que l'on utilise en principe deux largeurs de barres.

Fig. 1

Fig. 2

0 073 056

Fig. 4

Fig. 5a

Fig. 5b

Fig. 3

2